# EUROPEAN PATENT SPECIFICATION

(11) **EP 2 990 474 B1**
(45) Date of publication and mention of the grant of the patent: **07.06.2017**
(21) Application number: 15181615.4
(22) Date of filing: 19.08.2015
(51) Int. Cl.: C12N 1/12

(54) **A METHOD OF CULTIVATION OF SEA PROTIST BIOMASS, IN PARTICULAR OF MICROORGANISMS OF THE GENUS THRAUSTOCHYTRIALES**
KULTIVIERUNGSVERFAHREN VON MEERESEINZELLERBIOMASSE, INSBESONDERE VON MIKROORGANISMEN DER GATTUNG THRAUSTOCHYTRIEALES
PROCÉDÉ DE CULTURE DE BIOMASSE DE PROTISTES MARINS, NOTAMMENT DE MICRO-ORGANISMES DE L'ORDRE DES THRAUSTOCHYTRIALES

(30) Priority: 26.08.2014 CZ 20140576
(43) Date of publication of application: 02.03.2016
(73) Proprietor: Vysoká skola chemicko-technologická v Praze, 16628 Praha 6 (CZ); EcoFuel Laboratories s.r.o., 19000 Praha 9 (CZ)
(72) Inventor: Branyik, Tomas, 19000 Praha 9 (CZ); Humhal, Tomas, 25210 Lisnice (CZ); Kastanek, Petr, 12000 Praha 2 (CZ)
(74) Representative: Hartvichova, Katerina

(56) References cited:
- WO-A1-2005/045050
- WO-A1-2012/006307
- US-A- 5 246 842
- US-A1- 2006 094 089
- BONNET J L ET AL: "Biological treatment of whey by Tetrahymena pyriformis and impact study on laboratory-scale wastewater lagoon process", CHEMOSPHERE, vol. 38, no. 13, June 1999 (1999-06), pages 2979-2993, XP002752017, ISSN: 0045-6535
- Gernigon G. et al.: "Demineralization" In: Fuquay J.W. (Ed.): "Encyclopedia of dairy sciences (2nd ed,)", 2011, Elseviers Applied Sciences, London, Uk vol. 4, pages 2745-2751,

## Description

### Field of Art

The invention relates to a method of cultivation of sea protists, in particular of microorganisms of the genus *Thraustochytriales,* and to use of waste brine solution from the process of demineralization of sweet whey as a component of the cultivation medium for the purpose of biomass production. The thus obtained biomass is suitable as an additive into animal feed, food supplements or for use in pharmaceuticals and cosmetics.

### Background Art

Microorganisms of the genus *Thraustochytriales* (representatives of the order *Thraustochytriacae)* are saprophytic species occuring in seawater on the surface of algae, organic detritus or vascular plants. Typical representatives of this order include: *Aplanochytrium, Botryochytrium Japanochytrium, Schizochytrium, Sicyoidochytrium, Parietichytrium, Oblongichytrium, Ulkenia, Thraustochytrium.* From the biotechnological point of view, the *Thraustochytrids* are interesting microorganisms capable of effective accumulation of lipid fraction containing a major part of polyunsaturated fatty acids (PUFAs), in particular omega-3 fatty acids in their biomass. The PUFAs are essential components in human nutrition (Lewis T.E., Nicholas P.D., McMeekin T.A. (1999) The biotechnological potential of Traustochytrids. Marine Biotechnology 1: 580-587).

The most important omega-3 fatty acids are eicosapentaenoic acid (EPA) and docosahexaenoic acid (DHA). Clinical and epidemiological tests have shown medical effects of these acids; they positively affect namely cardiovascular system, central nervous system and immunity (Kris-Etherton P.M., Hecker K.D., Binkoski A.E. (2004) Polyunsaturated fatty acids and cardiovascular health. Nutr. Rev. 62:414-426).

The biotechnologically most interesting species are *Japanochytrium, Schizochytrium* and *Ulkenia,* as almost half of the lipid fraction usually consists of DHA (EP 1 685 255).

Typical cultivation media (such as ATCC 790 By+ medium) for the cultivation of the microorganisms of the genus *Thraustochytriales* are based on seawater, or a mixture of minerals mimicking the composition of seawater, a carbon source (in particular glucose or glycerol) and a source of nitrogen and vitamins (in particular yeast extract, peptone or corn extract). For instance, EP 1 685 255 discloses cultivation of the microorganism *Ulkenia* spec. strain SAM 2179 in a medium comprised of glucose, corn extract, KH₂PO₄, Na₂SO₄, MgCl₂, CaCl₂, (NH₄)₂SO₄ and CaCO₃.

Waste brine solution is obtained in the process of demineralization of sweet whey by electrodialysis in dairy factories. E.g., a dairy factory with a yearly production of ca 100 000 m³ of milk produces 30 - 60 m³ of waste brine solution per day. At present, no suitable use is proposed for this waste brine solution. This whey de-salting technology is used in tens of dairy factories worldwide, hence thousands of m³ of waste brine solution are produced daily. US2006/094089 discloses a method of culturing Thraustochytriales sp. in a medium where whey is the N-Source. US5246842 discloses a method of culturing Oomycetes on a fungal growth medium containing. lactose as a carbon source, obtained from a spray dried sweet whey powder, and/or demineralized whey, in particular dry sweet whey permeate.

### Disclosure of the Invention

The present invention provides a method of cultivation of sea protist biomass, in particular of microorganisms of the genus *Thraustochytriales,* wherein the cultivation is carried out in a cultivation medium comprising waste brine solution from the process of demineralization of sweet whey.

The waste brine solution from the process of demineralization of sweet whey may form at least 50 % v/v of the cultivation medium, preferably 50 to 98% v/v, or 50 to 95 % v/v of the cultivation medium. Preferably, it may be enriched with a source of carbon, typically glycerol or glucose; optionally also with a source of nitrogen and/or further growth-supporting substances, preferably selected from yeast extract, peptone and corn extract.

The sea protist biomass, in particular microorganisms of the genus *Thraustochytriales,* obtained according to the invention, may be used per se, or after extraction of unsaturated fatty acids, for the production of nutraceuticals, animal feed additives, pharmaceuticals or cosmetics.

The invention further encompasses use of waste brine solution from the process of demineralization of sweet whey as a component of a cultivation medium for cultivation of sea protist biomass, in particular of microorganisms of the genus *Thraustochytriales.*

Growth of model microorganisms *Schizochytrium limacinum* PA968 and *Japonochytrium sp.* AN4 was comparable on the waste brine medium and on the complex medium. The present invention thus provides a novel and economical method of cultivation of microorganisms of the genus *Thraustochytriales.*

The cultivation mediums for cultivation of sea protists, in particular of microorganisms of the genus *Thraustochytriales,* are generally comprised of a mixture of minerals mimicking the composition of seawater and at least one carbon source, optionally also nitrogen source. The invention is based on replacement of the seawater and partial replacement of the mixture of minerals and the nitrogen source by waste brine solution (WBS) produced in demineralization of sweet whey, said waste brine solution thus being a component of the cultivation medium for cultivation of microorganisms of the genus *Thraustochytriales.* A typical composition of the waste brine solution is shown in Table 1. The WBS solution must be neutralized for use for cultivation of microorganisms of the genus *Thraustochytriales* (pH adjustment from approx. 4.3 to 7 can be carried out using a suitable amount of NaOH) and it can be sterilized by means of membrane filtration or thermally. Sterile filtration is preferred, as it allows to maintain a bigger amount of carbon sources and organic substances in the medium. After neutralization and sterilization, a portion of the dissolved substances precipitates from the WBS (the precipitate may be removed by filtration); said precipitate contains all lactose from the WBS, and ca 90% of P, 95% of Ca, 60% of Na, 50% of Mg and 40 % of Cl from the WBS.

**Table 1 Chemical composition of the waste brine solution from demineralization of sweet whey (WBS) - example**

| Component | Value | Unit |
|---|---|---|
| Dry matter | 16.8 | g.kg-¹ |
| Ashes | 11.3 | g.kg-¹ |
| N_{Kjeldahl} | 0.2 | g.kg-¹ |
| Lactose | 3.0 | g.kg-¹ |
| Cl | 2.67 | g.kg-¹ |
| P | 0.64 | g.kg-¹ |
| SO₄²⁻ | 0.38 | g.kg-¹ |
| Ca | 0.79 | g.kg-¹ |
| Mg | 0.15 | g.kg-¹ |
| K | 3.32 | g.kg-¹ |
| Na | 3.32 | g.kg-¹ |
| NO³⁻ | 1.8 | g.kg-¹ |
| BSK₅ | 3.06 | g.kg-¹ |
| CHSK_{Cr} | 5.63 | g.kg-¹ |
| Conductivity | 15.0 | mS.cm⁻¹ |

| | | |
|---|---|---|
| BSK₅ = biological consumption of oxygen (5 days) CHSK_{Cr} = chemical consumption of oxygen determined by potassium dichromate | | |

### Brief Description of Drawings

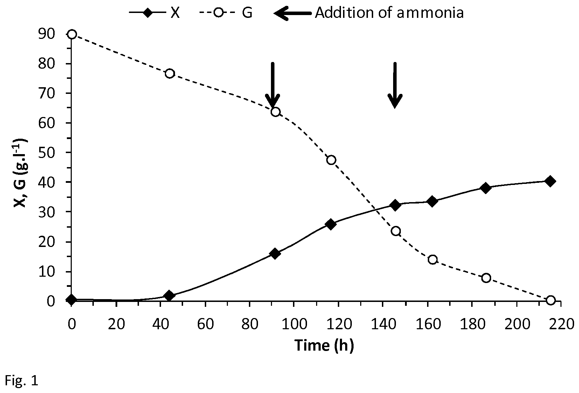

Fig. 1 represents the process of fermentation of *Schizochytrium limacinum* PA968 in a medium containing brine solution from demineralization of whey (WBS) with a repeated addition of ammonia. X - dry biomass concentration, G - glycerol concentration in the medium. An arrow designates the moment of addition of ammonia.

### Examples of carrying out the Invention

*Schizochytrium limacinum* PA968 and *Japonochytrium sp.* AN4 were used as model microorganisms of the genus *Thraustochytriales.*

### Example 1: Comparison of growth of Schizochytrium limaciuum PA968 in various media

From a Petri dish, the *Schizochytrium limacinum* PA968 cells were aseptically transferred into: 1) 100 ml of sterile complex medium (KM), or 2) 100 ml of sterile waste brine solution medium (SOM).

KM (pH 7) contained: glycerol (30 g.l-¹), yeast extract (10 g.l-¹), sodium chloride (18 g.l⁻¹), microelements (in mg.l⁻¹): 40 FeNa-EDTA, 88 CaCl₂, 0.83 H₃BO₃, 0.95 CuSO₄.5H₂O, 3.3 MnCl₂.4H₂O, 0.17 (NH4)₆Mo₇O₂₄.4H₂O, 2.7 ZnSO₄.7H₂O, 0.6 CoSO₄.7H2O, and 0.014 NH₄VO₃) and distilled water.

SOM (pH 7) was comprised of glycerol (30 g.l⁻¹) dissolved in thermally treated WBS. The cultures in Erlenmeyer flasks were placed into a shaker (130 rpm) at 23°C for 340 hours. The time course of the growth of *Schizochytrium limacinum* PA968 cultivated in KM and SOM is shown in Table 2.

**Table 2 Changes of concentration of dry biomass in the course of the growth of Schizochytrium limacinum PA968 on complex medium (KM) and on waste brine medium (SOM).**

| Cultivation time (h) | Biomass concentration (g.l⁻¹) | |
|---|---|---|
| | KM | SOM |
| 0 | 0.8 | 0.6 |
| 16 | 1.8 | 1.6 |
| 50 | 3.3 | 2.9 |
| 116 | 6.1 | 5.1 |
| 164 | 7.9 | 7.0 |
| 191 | 9.0 | 8.5 |
| 240 | 10.7 | 11.0 |
| 287 | 11.7 | 12.5 |
| 341 | 11.8 | 12.3 |

### Example 2: Comparison of growth of Japonochytrium sp. AN4 in various media

From a Petri dish, the *Japonochytrium sp.* AN4 cells were aseptically transferred into: 1) 100 ml of sterile complex medium (KM) and 2) 100 ml of sterile waste brine solution medium (SOM).

KM (pH 7) contained: glycerol (20 g.l⁻¹), yeast extract (10 g.l⁻¹), sodium chloride (18 g.l⁻¹), microelements (in mg l⁻¹: 40 FeNa-EDTA, 88 CaCl₂, 0.83 H₃BO₃, 0.95 CuSO₄.5H₂O, 3.3 MnCl₂.4H₂O, 0.17 (NH4)₆Mo₇O₂₄.4H₂O, 2.7 ZnSO₄.7H₂O, 0.6 CoSO₄.7H2O, a 0.014 NH₄VO₃) and distilled water.

SOM (pH 7) was comprised only from glycerol (20 g.l⁻¹) dissolved in thermally treated WBS. The cultures in Erlenmeyer flasks were placed into a shaker (130 rpm) at 23°C for 120 hours to cultivate the inoculum. Subsequently, the inocula were aseptically transferred into bioreactors Multifors (working volume 1 liter).

Complex medium (KM) in the bioreactor contained: glycerol (40 g.l⁻¹), yeast extract (20 g.l⁻¹), sodium chloride (18 g.l⁻¹), microelements (in mg l⁻¹: 40 FeNa-EDTA, 88 CaCl₂, 0.83 H₃BO₃, 0.95 CuSO₄.5H₂O, 3.3 MnCl₂.4H₂O, 0.17 (NH4)₆Mo₇O₂₄.4H₂O, 2.7 ZnSO₄.7H₂O, 0.6 CoSO₄.7H2O, a 0.014 NH₄VO₃) and distilled water.

Waste brine medium (SOM) in the bioreactor contained: glycerol (40 g.l⁻¹), yeast extract (20 g.l⁻¹) and thermally treated WBS.

Two parallel fermentations were performed for 103 hours at constant parameters: pH (7.0), stirring rate (180 min⁻¹), temperature (23°C), aeration (0.2 m³.h⁻¹.). The time course of the growth of *Japonochytrium sp.* AN4 in KM and SOM is shown in Table 3.

**Table 3 Changes of concentration of dry biomass in the course of the growth of Japonochytrium sp. AN4 on complex medium (KM) and on waste brine medium (SOM).**

| Cultivation time (h) | Biomass concentration (g.l⁻¹) | |
|---|---|---|
| | KM | SOM |
| 0 | 1.4 | 1.5 |
| 27 | 8.1 | 7.4 |
| 43 | 17.3 | 15.1 |
| 67 | 23.1 | 22.0 |
| 86 | 26.0 | 24.4 |
| 103 | 25.7 | 25.1 |

### Example 3: Cultivation of Schizochytrium limacinum PA968 in a medium containing waste brine solution (WBS) from demineralization of whey with repeated addition of ammonia

From a Petri dish, the *Schizochytrium limacinum* PA968 cells were transferred into 400 ml of sterile inoculation waste brine medium (SOM) which contained: glycerol (20 g.l⁻¹), yeast extract (10 g.l⁻¹) and thermally treated WBS. The cell suspension in Erlenmeyer flasks was placed into a shaker (130 rpm) at 23°C for 120 hours. Subsequently, the inoculum was aseptically transferred into a bioreactor Labfors (total volume 2.7 1). The starting composition of SOM in the bioreactor was: glycerol (90 g.l⁻¹), yeast extract (10 g.l⁻¹), ammonia (10 g.l⁻¹) and thermally treated WBS. Process parameters of the fermentation: working volume (2 1), inoculation (20 % vol.), pH (7.0), temperature (23°C), aeration (0.18 m³.h⁻¹), stirring (100 min⁻¹), oxygen transfer coefficient (k_{L}a = 72 h⁻¹). Ammonia was added in two portions during the cultivation (each portion was 5 g.l⁻¹). The course of the fermentation of *Schizochytrium limacinum* PA968 in the medium containing waste brine from the demineralization of whey (WBS) with the repeated addition of ammonia is shown in Fig. 1 and the results are shown in Table 4.

**Table 4 Results of fermentation and composition of dry biomass of Schizochytrium limacinum PA968**

| Parameter | value | |
|---|---|---|
| Dry biomass concentration | 40.4 | g.l⁻¹ |
| CMK^{a} in biomass | 19.6 | % hm. |
| CMK^{a} in medium | 7.9 | g.l⁻¹ |
| Ratio of DHA in CMK^{a} | 48.5 | % hm. |
| Concentration of DHA in medium | 3.8 | g.l⁻¹ |
| Biomass productivity | 4.51 | g.l⁻¹.day⁻¹ |
| Productivity DHA | 424 | mg.l⁻¹.day⁻¹ |

| | | |
|---|---|---|
| ^{a} total fatty acids | | |

### Industrial Applicability

The invention is usable in biotechnology industry. The process of the invention can be performed using the current industrial fermentation capacities. The *Thraustochytriales* microorganism biomass represents a valuable raw material for many popular products in foodstuff and pharmaceutical industry, in cosmetics, as a component of food supplements, animal feeds, unsaturated omega-3 fatty acids and other products.

## Claims

1. A method of cultivation of sea protist biomass, in particular of microorganisms of the genus *Thraustochytriales,* **characterized in that** the cultivation is carried out in a cultivation medium comprising waste brine solution from the process of demineralization of sweet whey.

2. The method according to claim 1, wherein the content of the waste brine solution from the process of demineralization of sweet whey in the cultivation medium is at least 50 % v/v.

3. The method according to claim 1 or 2, wherein a carbon source, preferably selected from glycerol and glucose, is dissolved in the waste brine solution from the process of demineralization of sweet whey.

4. The method according to any one of claims 1 to 3, wherein a source of nitrogen and/or growth-supporting substances, preferably selected from yeast extract, peptone and corn extract, is dissolved in the waste brine solution from the process of demineralization of sweet whey.

5. Use of waste brine solution from the process of demineralization of sweet whey as a component of a cultivation medium for cultivation of sea protist biomass, in particular of microorganisms of the genus *Thraustochytriales,*

## Patentansprüche

1. Verfahren zur Kultivierung von Meereseinzellerbiomasse, insbesondere von Mikroorganismen der Gattung *Thraustochytriales,* **dadurch gekennzeichnet, dass** die Kultivierung in einem Kultivierungsmedium durchgeführt wird, das Abfall-Sole-Lösung aus dem Prozess der Demineralisierung von Süßmolke umfasst.

2. Verfahren nach Anspruch 1, **dadurch gekennzeichnet, dass** der Gehalt der Abfall -Sole-Lösung aus dem Prozess der Demineralisierung von Süßmolke im Kultivierungsmedium mindestens 50 % v/v beträgt.

3. Verfahren nach Anspruch 1 oder 2, **dadurch gekennzeichnet, dass** eine Kohlenstoffquelle, vorzugsweise ausgewählt aus Glycerin und Glucose, in der Abfall -Sole-Lösung aus dem Prozess der Demineralisierung von Süßmolke gelöst wird.

4. Verfahren nach einem der Ansprüche 1 bis 3, **dadurch gekennzeichnet, dass** eine Stickstoffquelle und/oder wachstumsfördernden Substanzen, vorzugsweise ausgewählt aus Hefeextrakt, Pepton und Maispflückstoff, in der Abfall -Sole-Lösung aus dem Prozess der Demineralisierung von Süßmolke gelöst wird.

5. Verwendung von Abfall-Sole-Lösung aus dem Prozess der Demineralisierung von Süßmolke als Bestandteil eines Kultivierungsmediums für die Kultivierung von Meereseinzellerbiomasse, insbesondere von Mikroorganismen der Gattung *Thraustochytriales.*

## Revendications

1. Procédé de culture de la biomasse des protistes marins, notamment de microorganismes de l'ordre des *Thraustochytriales,* **caractérisé en ce que** la culture est réalisée dans un milieu de culture comprenant une solution de saumure résiduelle du procédé de déminéralisation du lactosérum doux.

2. Procédé selon la revendication 1, dans lequel le contenu de la solution de saumure résiduelle du procédé de déminéralisation du lactosérum doux dans le milieu de culture est d'au moins 50 % v/v.

3. Procédé selon la revendication 1 ou 2, dans lequel une source de carbone, de préférence choisie parmi le glycerol et le glucose, est dissoute dans la solution de saumure résiduelle du procédé de déminéralisation du lactosérum doux.

4. Procédé selon l'une quelconque des revendications 1 à 3, dans lequel une source d'azote et/ou substances supportant la croissance, de préférence choisies parmi l'extrait de levure, la peptone et l'extrait de maïs, est dissoute dans la solution de saumure résiduelle du procédé de déminéralisation de lactosérum doux.

5. Utilisation de la solution de saumure résiduelle du procédé de déminéralisation du lactosérum doux en tant que composante d'un milieu de culture pour la culture de la biomasse des protistes marins, notamment de microorganismes de l'ordre des *Thraustochytriales.*
